# EUROPEAN PATENT SPECIFICATION

(11) **EP 4 271 338 B1**
(45) Date of publication and mention of the grant of the patent: **10.12.2025**
(21) Application number: 20841952.3
(22) Date of filing: 29.12.2020
(51) Int. Cl.: A61F 13/47, A61F 13/475, A61F 13/533

(54) **SANITARY ARTICLE WITH EMBOSSED FUNCTIONAL DESIGN ELEMENTS**
HYGIENEARTIKEL MIT GEPRÄGTEN FUNKTIONSDESIGNELEMENTEN
ARTICLE D'HYGIÈNE AVEC ÉLÉMENTS GAUFRÉS DE CONCEPTION FONCTIONNELLE

(43) Date of publication of application: 08.11.2023
(73) Proprietor: Essity Hygiene and Health Aktiebolag, 405 03 Göteborg (SE)
(72) Inventor: DAHLBERG, Johan, 405 03 Göteborg (SE); NYSTRÖM, Anna-Karin, 405 03 Göteborg (SE)
(74) Representative: Nederlandsch Octrooibureau
(86) International application number: PCT/EP2020/087974
(87) International publication number: WO 2022/144074

(56) References cited:
- US-A1- 2017 246 045
- US-A1- 2018 360 672
- US-A1- 2020 360 195
- US-B2- 10 376 424
- US-B2- 9 040 769

## Description

### TECHNICAL FIELD

The present disclosure pertains to a sanitary article such as a sanitary napkin or a pantiliner. In particular, this disclosure pertains to a sanitary article having embossed functional design elements.

### BACKGROUND ART

Sanitary articles to which this disclosure relates are typically intended to absorb body liquids, such as urine and blood. Users put high demands on such articles, requiring them to be thin, comfortable and at the same time effectively absorb body liquids.

Disposable sanitary articles, such as sanitary napkins, pantyliners or the like typically include a liquid pervious topsheet, intended to face the wearer during use, a liquid impervious backsheet and an absorbent structure therebetween. Such absorbent structures may be relatively thin and compressed. Absorbent structures that are relatively compressed may lack flexibility and it is important to ensure that these relatively thin and compressed articles absorb liquid quickly, do not leak and are comfortable to wear. One possible solution is to shape the article to have a bowl-shaped structure, such as with channels and elastic elements. However, bowl-shaped structures may be bulky and less body conforming and thus less discreet. For feminine sanitary napkins, the fit in each of the front, crotch and rear portions is important for preventing leakage but also for the sanitary article to be discreet and comfortable during use.

In one example, US 2020/0360195 provides a disposable absorbent article having embossed central channels disposed in the central region of the article said to contribute to retaining absorbed bodily fluids and to an increase in conformance of the article.

It is furthermore of importance that the sanitary article gives the user a visual perception of leakage security and good fit, enabling the user to feel secure and thereby enhancing the well-being of the user.

In view of the above, there is a need for a sanitary article which has a rapid intake, good fit, is discreet to wear and has an enhanced visual appearance.

### SUMMARY

One or more of the above objects is achieved with a sanitary article according to claim 1, Further advantages and advantageous features of the invention are disclosed in the following description and in the dependent claims.

A sanitary article, being a sanitary napkin or a pantiliner, comprises a topsheet, a backsheet and an absorbent core arranged between the topsheet and the backsheet. The sanitary article has an extension in a longitudinal direction and in a transverse direction. The sanitary article furthermore has a first and a second longitudinal side edge extending in the longitudinal direction and a front and a rear transverse side edge extending in the transverse direction. The sanitary article is made up of a front portion, a crotch portion and a rear portion, as seen in the longitudinal direction. The absorbent core comprises a head portion, a body portion and a neck portion, the neck portion forming a transitional region between the head portion and a body portion. The head portion is positioned in the front portion of the sanitary article and the body portion is positioned in the crotch portion and in the rear portion of the sanitary article. A transverse width of the absorbent core at the neck region is smaller than a maximum transverse width of the absorbent core in the head portion and in the body portion. The absorbent core furthermore comprises three individual and separate embossed functional design elements, an embossed front functional design element being arranged in the front portion of the sanitary article, an embossed crotch functional design element being arranged in the crotch portion of the sanitary article and an embossed rear functional design element being arranged in the rear portion of the sanitary article. Each of the embossed functional design elements has a boundary comprising continuous or discontinuous line(s) delimiting a respective inner region of the embossed functional design element and wherein each of the embossed front, crotch and rear functional design elements is different from one another in shape and/or size.

The term "sanitary article" refers to a product that is placed against the skin of the wearer in the wearer's crotch to absorb and contain body exudates, such as urine, faeces and menstrual fluid. The disclosure mainly relates to disposable sanitary articles, which means articles that are not intended to be laundered or otherwise restored or reused as a sanitary article. Examples of disposable sanitary articles include feminine hygiene products such as sanitary napkins, panty liners, incontinence pads and the like.

The front portion, crotch portion and rear portion may be of equal length as seen in the longitudinal direction. Articles especially used during night are often longer to better protect the user from leakage when lying down. The proportion between the different portions could alternatively be that the crotch and the rear portion may constitute 40% each, as seen in the longitudinal direction, of the length of the sanitary article and the front portion may constitute 20% of the length of the sanitary article, as seen in the longitudinal direction. The measurements being made from the frontmost and the rearmost point of the sanitary article and being measured in the longitudinal direction.

Each of the embossed functional design elements has a boundary comprising continuous or discontinuous line(s) delimiting a respective inner region of the embossed functional design elements, the discontinuous line may be a dotted line or a dashed line, or a combination thereof. The spaces between the dots or the dashes may be equal to or smaller than the dots or dashes, as seen in the extension of the discontinuous line.

The embossings of the respective functional design elements may be made by embossing the topsheet and the absorbent core together or by providing embossings to only the absorbent core.

The transverse width of the absorbent core at the neck region may be smaller than any transverse width of the head portion of the absorbent core, the width being measured along longitudinal side edges of the absorbent core and does not include configurations of the core in which the head portion is rounded at a front edge of the absorbent core. The transverse width of the neck region may be smaller than any transverse width of the body portion of the absorbent core, the width being measured along longitudinal side edges of the absorbent core and does not include configurations of the core in which the body portion of the is rounded at a rear edge of the absorbent core.

With the sanitary article according to the present disclosure, the absorbent core has a neck region arranged at the groin area of the user where compressive forces is received in a lateral direction. The provision of a neck region retains the flat shape of the absorbent core in this area and reduces twists or wrinkles which may otherwise arise and the sanitary article will better be held in its position during the use. Such configuration furthermore means that the article can bend in a transversal direction more easily in the area of the neck portion. Therefore, the front portion and the core head portion can bend towards the user and thus the front portion can better cover the pubic regions of the wearer while the crotch portion is able to be positioned close to the crotch area of a wearer. For an enhanced fit, individual and predictable shaping of the sanitary napkin in each of the front, crotch and rear portions are important. This prevents leakage but is also of importance for the sanitary article to be discreet and comfortable during use. Each of these portions differs from each other, both in terms of absorbency needs and the shaping needed to fit the user anatomy in the respective region.

It is furthermore of importance that the sanitary article gives the user a visually perception of leakage security and good fit, enabling the user to feel secure and thereby enhancing the well-being of the user. The provision of an embossed functional design element in the respective front, crotch and rear regions which is different from the embossed functional design element in the other regions allows customization of the embossed functional design elements. Hence, the predictable folding and shaping, in particularly the front and rear regions, provided by the functional design elements may be adapted to the anatomy of the user in these regions. The embossed functional design element arranged in the crotch region may be provided to provide leakage security and a visual cue for the user of leakage security. Additionally, the front and rear embossed functional design elements may function as visual cues for the user of the intended folding and shaping, thereby indicating how the sanitary article should be shaped when putting the sanitary article in the underwear, thus providing a slight pre-shaping of the sanitary article and thus improving the fit of the sanitary napkin during use. The absorbent core may comprise a higher amount of absorbent material at the crotch embossed functional design element compared to at the front and/or rear embossed function design element, such as a higher amount of superabsorbent polymer and/or cellulosic fibers. The absorbent core at the crotch embossed functional design element may optionally have at least 20% or 30% or 50% higher amount of absorbent material, as measured in g/m² and as a mean value within the region of the respective embossed function design element.

Each of the front, the crotch and the rear functional design elements are different from one another in shape and/or size. The front, crotch or rear embossed functional design element are different in shape and/or size to provide customized functionality in the respective area. The embossed crotch functional design element may for example be larger in size than the respective embossed front and rear functional design element and be designed to enhance the liquid intake and to reduced liquid spreading to the front and rear portions of the sanitary article. The inner region of the embossed crotch functional design element may for example be at least 25%, 30%, 40% or 50% greater in size than the inner region of the embossed rear functional design element. The inner region of the embossed crotch functional design element may for example be at least 30%, 40%, 50% or 60% greater in size than the inner region of the embossed front functional design element.

The sanitary article may have a first fastening wing extending along the first longitudinal side edge between a first front wing junction and a first rear wing junction and a second fastening wing extending along the second longitudinal side edge between a second front wing junction and a second rear wing junction. The front and rear wing junction correspond to a front and rear deflection point, as taken from the longitudinal side edge.

The first front wing junction and/or the second front wing junction may be aligned with the neck region of the absorbent core, as seen in the longitudinal direction, or may be arranged not more than 30 mm rearwardly from the neck region as measured in the longitudinal direction, optionally not more than 15 mm rearwardly from the neck region as measured in the longitudinal direction.

The embossed front functional design element may have a V-shaped section pointing towards the rear portion of the sanitary article and wherein a tip of the V-shaped section of the embossed front element may be arranged not more than 30 mm, preferably not more than 15 mm or not more than 10 mm, from the neck region, as measured in the longitudinal direction. The fact that the embossed front functional design element has a tapering shape with the tip of the V-shaped section accentuating the neck region may promote a desired upwards, and slightly inwardly, flexing of the front portion, thereby conforming to the user body. The V-shaped section may also function as a stiffening element. This arrangement of the V-shaped section of the embossed front functional design element has been found to improve correct body conforming shaping of the sanitary article over time as the article is being worn. This may prevent the sanitary article from folding in an undesired manner due to movement or due to malpositioning when the user attaches the article in the undergarment. The embossed front functional design element may for example be in the form of a heart, or any other shape having a V-shaped section.

The embossed crotch functional design element may comprise a first and a second longitudinally extending side section and a distance between the first and the second longitudinally extending side sections may increase rearwardly, as seen in the longitudinal direction. Since the sanitary napkin is intended to be shaped to have close fit to the user body, it has found to be of particular benefit to have embossed longitudinally extending side sections which steer the liquid into the absorbent core and reduce or eliminate side leakage. The distance between the first and second longitudinally extending side section may for example vary between at least 30% of the width of the absorbent core to at least 50%, 60% or 70% of the width of the absorbent core, as measured in the transverse direction and at the same transverse location as the distance is measured. The increase in distance between the first and a second longitudinally extending side section may correlate to an increase in width of the body portion of the absorbent core.

The embossed crotch functional design element may have a front V-shaped section pointing towards the front edge of the sanitary article and/or a rear V-shaped section pointing towards the rear edge of the sanitary article.

The front and/or rear V-shaped sections of the embossed crotch functional design element may be connected to the first and second longitudinally extending side sections.

The V-shaped section of the embossed front functional design element and the front V-shaped section of the embossed crotch functional design element may be arranged with a distance to each other of between 2 and 15 mm, as seen in the longitudinal direction. This further improves correct body conforming shaping of the sanitary article over time as the article is being worn.

The first and the second longitudinally extending side sections of the embossed crotch functional design element may have a respective first and second rear side section end point and the first and/or second rear side section end point may be aligned with or may be arranged not more than 30 mm, optionally not more than 15 mm, from the first and/or second rear wing junction. The first and second longitudinally extending side sections of the embossed crotch functional design element may have a respective first and second front side section end point and wherein the first and/or second rear side section end point may be aligned with or may be arranged not more than 30 mm, optionally not more than 15 mm, from the first and/or second front wing junction. The first and the second longitudinally extending side sections of the embossed crotch functional design element may thereby function to provide both stabilization and leakage security in a region where the first and second fastening wings stretch the sanitary laterally outwardly.

The absorbent core may have a greater absorbent capacity in the inner region of the embossed crotch functional design element than within the inner region of the embossed front functional design element and/or the embossed rear functional design element.

The embossed rear functional design element may have a first V-shaped section pointing rearwardly of the sanitary article. The first V-shaped section may be arranged with a point of the V-shaped section being aligned with a longitudinal centerline of the sanitary article. This may facilitate predictable inward folding of the sanitary article along a longitudinal centerline of the sanitary article in the buttocks region, thus conforming the sanitary article to the user anatomy and providing a discreet and body conforming sanitary article.

The embossed rear functional design element may have a second V-shaped section pointing towards the front portion of the sanitary article and the second V-shaped section may be provided closer to the rear transverse edge of the sanitary article than the first V-shaped section of the embossed rear functional design element. The first and the second V-shaped sections of the embossed rear functional design element may be connected with a first and a second longitudinally extending side section of the embossed rear functional design element. The first and second V-shaped sections of the embossed rear functional design element may be aligned with each other and optionally with a longitudinal centreline of the sanitary article.

The inner region of the embossed rear functional design element may be provided with a first and a second embossed line and the first and the second embossed line may extend in parallel with a respective first and second leg section of the first V-shaped section of the embossed rear functional design element.

The inner region of the embossed rear functional design element may be provided with a third and a fourth embossed line and wherein the third and the fourth embossed line extend in parallel with a respective leg section of the first V-shaped section of the embossed rear functional design element, such that the first and the third embossed line are parallel with the first leg section and the second and the fourth leg sections are parallel with the second leg section of the first V-shaped section of the embossed rear functional design element. The embossed lines may be slightly curved, with a main portion extending in parallel with the respective first and second leg section of the first V-shaped section of the embossed rear functional design element.

The embossed lines provided in the inner region of the embossed rear functional design element may further enhance the shaping of the rear portion of the sanitary article provided by the embossed rear functional design element.

A tip of the rear V-shaped section of the embossed crotch functional design element may be arranged with a distance of between 2 mm and 20 mm from a tip of the first V-shaped section of the embossed rear functional design element, optionally wherein a first and a second leg section of the rear V-shaped section of the embossed crotch functional design element extend in parallel with a first and a second leg section of the first V-shaped section of the embossed rear functional design element.

Each of the embossed front functional design element, the embossed crotch functional design element and the embossed rear functional design element may be aligned with each other, as seen in the longitudinal direction, and be centred along a longitudinal centerline of the sanitary article.

The fastening wings may be provided with attachment means, such as adhesive, for attaching the fastening wings to an undergarment. The fastening wings are intended to be folded around the undergarment and attached to be attached the undergarment garment on a garment-facing side thereof.

The sanitary article according to the present disclosure may be provided with an adhesive, such as a pressure-sensitive adhesive on the backsheet. The adhesive may be covered with an elongated wrapping sheet being releasably adhered the adhesive-bearing backsheet of the sanitary article. The wrapping sheet may be coated with material which enables the release of the wrapping sheet, such as silicone, so that the wrapping sheet may be peeled away from the adhesive-bearing backsheet of the sanitary article and so that the sanitary article may be attached to an undergarment. The adhesive-bearing region of the backsheet may also be covered with a release paper. The release paper may be attached to the wrapping sheet, for example with a permanent attachment, such that the sanitary article is attached to the wrapping sheet via the release paper.

### BRIEF DESCRIPTION OF THE DRAWINGS

The present invention will be further explained hereinafter by means of nonlimiting examples and with reference to the appended drawings wherein:
Fig. 1 illustrates a sanitary article according to the present disclosure;
Fig. 2 illustrates an alternative sanitary article according to the present disclosure; and
Fig. 3 illustrates a sanitary article with the definition of the front wing junctions and the rear wing junctions.

### DETAILED DESCRIPTION

It is to be understood that the drawings are schematic and that individual components are not necessarily drawn to scale. The sanitary articles shown in the figures are provided as examples only and should not be considered limiting to the invention. Accordingly, the scope of invention is determined solely by the scope of the appended claims.

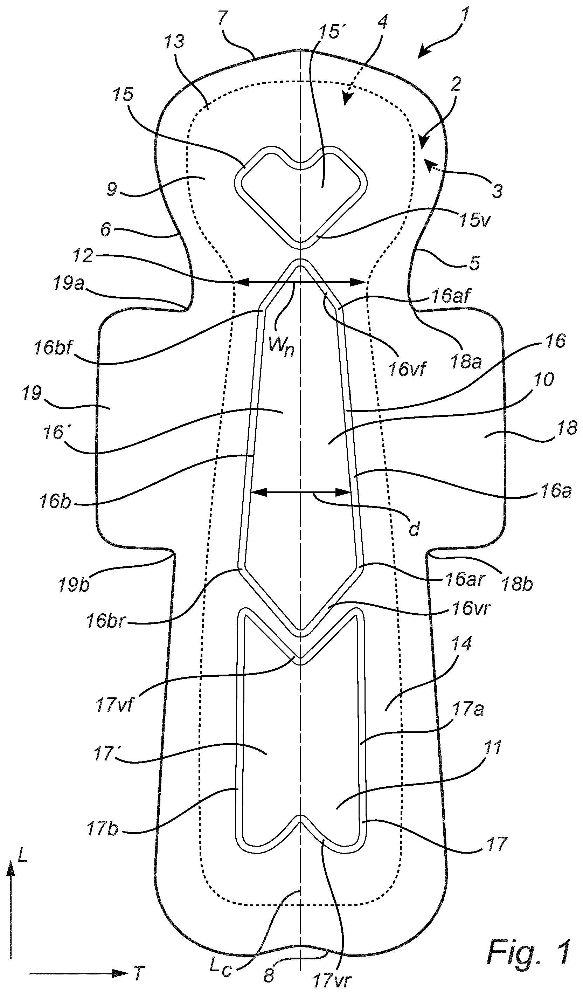

Fig. 1 illustrates a sanitary article 1 according to the present disclosure. The sanitary article 1 comprises a topsheet 2, a backsheet 3 and an absorbent core 4 arranged between the topsheet 2 and the backsheet 3.

The topsheet 2 may include or consist of fibrous nonwoven layer(s) being spunbonded, meltblown, carded, hydroentangled, wetlaid. Suitable nonwoven materials can be composed of natural fibers, such as woodpulp or cotton fibers, synthetic thermoplastic fibers, such as polyolefins, polyesters, polyamides and blends and combinations thereof or from mixtures of natural and synthetic fibers. The materials suited as topsheet material should be soft and non-irritating to the skin and be readily penetrated by body fluid, such as menstrual fluid and urine.

The backsheet 3 may consist of a thin plastic film, e.g. a polyethylene or polypropylene film, a nonwoven material coated with a liquid impervious material, a hydrophobic nonwoven, which resist liquid penetration. Laminates of plastic films and nonwoven materials may also be used. The backsheet material can be breathable to allow vapor to escape from the absorbent structure, while still preventing liquids from passing through the backsheet material.

The absorbent core 4 is the absorbent structure of the article which acquires and stores bodily fluids. The absorbent core may be of any conventional kind. Examples of commonly occurring absorbent materials are cellulosic fluff pulp, tissue, highly absorbent polymers (so called superabsorbents), absorbent foam materials, absorbent nonwoven materials or the like. It is common to combine cellulosic fluff pulp with superabsorbent polymers in an absorbent core. Superabsorbent polymers are water-swellable, water-insoluble organic or inorganic materials capable of absorbing at least about 20 times their own weight of an aqueous solution containing 0.9 weight percent of sodium chloride. Organic materials suitable for use as a superabsorbent material can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyacrylamides, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fibre, flake, sphere, etc. possesses a higher crosslink density than the inner portion of the superabsorbent. The superabsorbent materials may be in any form suitable for use in absorbent composites including particles, fibres, flakes, spheres, and the like. A high absorption capacity is provided by the use of high amounts of superabsorbent material. Thin absorbent cores which are common in for example sanitary napkins, baby diapers and incontinence guards, often comprise a compressed, mixed or layered structure of cellulosic fluff pulp and superabsorbent polymers. The size and absorbent capacity of the absorbent core may be varied to suit different product types, such as sanitary napkins for adult incontinent persons or panty liners.

Generally, the core can be of unitary construction, whereby for example the manufacturing process can be simplified. The phrase "unitary construction" in the present context is intended to mean that the absorbent core is constructed from essentially one type of material, this being essentially the same material, or essentially the same combination of two or more materials throughout the absorbent core. Variations in density and concentration of the material may occur, but these are limited to those which may be obtained without incorporation of regions which have been formed separately and then physically joined to each other. For example, when the absorbent core comprises a matrix of hydrophilic fibres and superabsorbent material as described above, the relative concentrations of superabsorbent material and fibres may be different in different parts of the core. However, the absorbent core of unitary construction does not comprise layers or laminates of different composition. Likewise, variations in the density or concentration of various components across the longitudinal direction, the transverse direction or the thickness direction of the absorbent core are acceptable, yet the core should not comprise areas or layers of different composition which are formed separately and later joined together.

A suitable technique for manufacturing the absorbent cores of the present disclosure is mat- forming through an air-laying process. In the process an airpermeable mould is provided. Fibrous material is air-laid into the mould and the mould is filled, whereby an absorbent core is produced in with a desired amount of fibrous material.

The sanitary article 1 has an extension in a longitudinal direction L and in a transverse direction T. The sanitary article 1 has first and second longitudinal side edges 5,6 extending in the longitudinal direction L and front and rear transverse side edges 7,8 extending in the transverse direction T. The sanitary article is made up of a front portion 9, a crotch portion 10 and a rear portion 11, as seen in the longitudinal direction L. The absorbent core 4 comprises a head portion 13, a body portion 14 and a neck portion 12, the neck portion 12 forming a transitional region between the head portion 13 and the body portion 14, with the head portion 13 being positioned in the front portion 9 of the sanitary article 1 and the body portion 14 being positioned in the crotch portion 10 and in the rear portion 11 of the sanitary article 1. A transverse width wₙ of the absorbent core 3 at the neck region 12 is smaller than a maximum transverse width of the absorbent core 4 in the head portion 13 and in the body portion 14.

The sanitary article 1 has a first fastening wing 18 extending along the first longitudinal side edge 5 between a first front wing junction 18a and a first rear wing junction 18b and a second fastening wing 19 extending along the second longitudinal side edge 6 between a second front wing junction 19a and a second rear wing junction 19b. The first front wing junction 18a and the second front wing junction 19a are arranged not more than 15 mm rearwardly from the neck region 12 as measured in the longitudinal direction L.

In accordance with the present disclosure, the absorbent core 4 is embossed with functional design elements and comprises three individual and separate embossed functional design elements 15,16,17. The respective functional design elements 15,16,17 are made by embossing of the topsheet 2 and the absorbent core 4 together. However, the embossings may alternatively be made in only the absorbent core 4. The embossed functional design elements 15,16,17 having a boundary comprising continuous or discontinuous line(s) delimiting a respective inner region 15',16,'17' of the embossed functional design elements 15,16,17. In Fig. 1, each of the embossed front, crotch and rear functional design elements 15,16,17 is different from one another in both size and shape. The inner region of the embossed crotch functional design element may for example be at least 25%, 30%, 40% or 50% greater in size than the inner region of the embossed rear functional design element. The inner region of the embossed crotch functional design element may for example be at least 30%, 40%, 50% or 60% greater in size than the inner region of the embossed front functional design element. The absorbent core may furthermore comprise a higher amount of absorbent material at the crotch embossed functional design element compared to at the front and/or rear embossed function design element, such as a higher amount of superabsorbent polymer and/or cellulosic fibers.

An embossed front functional design element 15 is arranged in the front portion 9 of the sanitary article 1. The embossed front functional design element 15 has a heart shape including a V-shaped section 15v pointing towards the rear portion 11 of the sanitary article 1. A tip of the V-shaped section 15v of the embossed front element 15 is arranged not more than 15 mm, preferably not more than 10 mm, from the neck region 12, as measured in the longitudinal direction L.

An embossed crotch functional design element 16 is arranged in the crotch portion 10 of the sanitary article 1. The embossed crotch functional design element 16 comprises a front V-shaped section 16vf pointing towards the front edge 7 of the sanitary article 1 and a rear V-shaped section 16vr pointing towards the rear edge 8 of the sanitary article 1. A first and a second longitudinally extending side section 16a, 16b extends between and connects the front V-shaped section 16vf and the rear V-shaped section 16vr of the embossed crotch functional design element 16. As may be seen in Fig. 1, a distance d between the first and the second longitudinally extending side sections 16a,16b increases rearwardly, as seen in the longitudinal direction L. The V-shaped section 15v of the embossed front functional design element 15 and the front V-shaped section 16vf of the embossed crotch functional design element 16 are arranged at a distance to each other of between 2 and 20 mm, as seen in the longitudinal direction L.

The first and the second longitudinally extending side sections 16a, 16b of the crotch embossed functional design element 16 have a respective first and second rear side section end point 16ar, 16br, the first and second rear side section end point being arranged not more than 15 mm from the first and/or second rear wing junction 18b,19b, as seen in the longitudinal direction L.

An embossed rear functional design element 17 is arranged in the rear portion 11 of the sanitary article 1. The embossed rear functional design element 17 has a first V-shaped section 17vf pointing rearwardly of the sanitary article 1 and a second V-shaped section 17vr pointing towards the front portion of the sanitary article 1. The second V-shaped section 17vr of the embossed rear functional design element 17 is provided closer to the rear transverse edge 8 of the sanitary article 1 than the first V-shaped section 17vf of the embossed rear functional design element 17. The first and the second V-shaped sections 17vf,17vr of the embossed rear functional design element 17 are connected with a first and a second longitudinally extending side section 17a, 17b of the embossed rear functional design element 17.

Each of the embossed front functional design element 15, the embossed crotch functional design element 16 and the embossed rear functional design element 17 is aligned with each other, as seen in the longitudinal direction L, and centred along a longitudinal centerline L_{C} of the sanitary article 1.

The sanitary article 1 according to the present disclosure may further include a liquid acquisition sheet (not shown in the Figs.), which acts as a liquid distribution layer. The acquisition layer can have different shapes which may be adapted to suit the shape of the absorbent core. The acquisition layer may extend 1-2 mm or more beyond the outer edge of the core 4 (preferably around the entire periphery of the core 4). This configuration can provide neat edges at the periphery of the article. The liquid acquisition sheet may be located between the topsheet 2 and the core 4 and is suitably placed on top of the absorbent core. The liquid acquisition sheet is adapted to quickly receive and temporarily store discharged liquid before it is absorbed by the absorbent core 6. Such acquisition distribution layers may be composed of for example airlaid nonwoven, spunlace nonwoven, high loft nonwoven or foam materials. The nonwoven material may be hydrophilic. A hydrophilic material may be obtained by adding a surfactant.

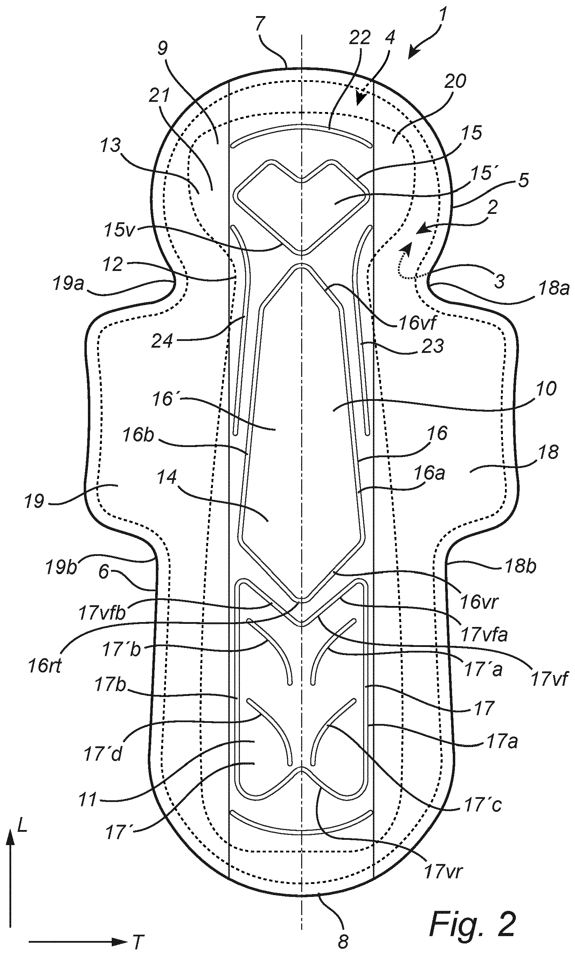

Fig. 2 illustrates a second embodiment of a sanitary article 1 according to the present disclosure. The same reference signs as in Fig. 1 are used to denote the same or similar features. Thus, the sanitary article comprises a topsheet 2, a backsheet 3 and an absorbent core 4 arranged between the topsheet 2 and the backsheet 3. The sanitary article 1 has first and second longitudinal side edges 5,6 extending in the longitudinal direction L and front and rear transverse side edges 7,8 extending in the transverse direction T. The sanitary article is made up of a front portion 9, a crotch portion 10 and a rear portion 11, as seen in the longitudinal direction L. The absorbent core 4 comprises a head portion 13, a body portion 14 and a neck portion 12, the neck portion 12 forming a transitional region between the head portion 13 and the body portion 14, the head portion 13 being positioned in the front portion 9 of the sanitary article 1 and the body portion 14 being positioned in the crotch portion 10 and in the rear portion 11 of the sanitary article 1. The topsheet 2 of the sanitary article 1 being covered with textile side edges 20,21 on a first and second longitudinal edge portion of the sanitary article to provide an enhanced sensorial effect for the user and to steer the liquid intake to the crotch portion 10 of the sanitary article 1. The textile side edges 20,21 may for example be a polypropylene carded thermobonded hydrophilic nonwoven (fibrous) material. Also, spunbond nonwoven, an air-thru bonded nonwoven, a spunlaced (hydroentangled) nonwoven, a meltblown nonwoven, or a combination of these can be used. If a combination is used, there can be a mixture of fibers from different polymers, but each fiber can also consist of different polymers (For example: bicomponent fibers PP/PE or copolymer PP/PE). The textile edge nonwoven can also include a percentage of natural fibers, such as pulp or viscose. The nonwoven can be hydrophilic, permanent hydrophilic or hydrophobic. The nonwoven can have a basis weight of 7 to 50 gsm. Also, the material in the textile edges can be a plastic film, made of PP, PE, PET, PLA, starch or any other thermoplastic polymer, or a blend or a copolymer of the polymers mentioned. The material can also be a laminate of a nonwoven and a film. Such a laminate can be made by bonding the nonwoven and the film by using heat, by using an adhesive, by mechanical bonding or by extrusion of the film on the nonwoven, or a combination of these methods. The textile side edges 20,21 may for example extend on 10 to 35 mm from the longitudinal side edges 5,6 of the sanitary article 1.

The sanitary article 1 has a first fastening wing 18 extending along the first longitudinal side edge 5 between a first front wing junction 18a and a first rear wing junction 18b and a second fastening wing 19 extending along the second longitudinal side edge 6 between a second front wing junction 19a and a second rear wing junction 19b. The first front wing junction 18a and the second front wing junction 19a are arranged not more than 15 mm rearwardly from the neck region 12 as measured in the longitudinal direction L.

The absorbent core 4 is embossed with functional design elements and comprises three individual and separate embossed functional design elements 15,16,17. The respective functional design elements 15,16,17 are made by embossing of the topsheet 2 and the absorbent core 4 together. However, the embossings may alternatively be made in only the absorbent core. The embossed functional design elements 15,16,17 having a boundary comprising continuous or discontinuous line(s) delimiting a respective inner region 15',16,'17' of the embossed functional design elements 15,16,17 and each of the embossed front, crotch and rear functional design elements 15,16,17 is different from one another in both size and shape.

An embossed front functional design element 15 is arranged in the front portion 9 of the sanitary article 1. The embossed front functional design element 15 has a heart shape including a V-shaped section 15v pointing towards the rear portion 11 of the sanitary article 1. A tip of the V-shaped section 15v of the embossed front element 15 is arranged not more than 15 mm, preferably not more than 10 mm, from the neck region 12, as measured in the longitudinal direction L. An embossed curved line 22 is provided frontwards of the embossed front functional design element 15, essentially following the curvature of the front edge 7 of the absorbent core 4.

An embossed crotch functional design element 16 is arranged in the crotch portion 10 of the sanitary article 1. The embossed crotch functional design element 16 comprises a front V-shaped section 16vf pointing towards the front edge 7 of the sanitary article 1 and a rear V-shaped section 16vr pointing towards the rear edge 8 of the sanitary article 1. A first and a second longitudinally extending side section 16a, 16b extends between and connects the front V-shaped section 16vf and the rear V-shaped section 16vr of the embossed crotch functional design element 16. As may be seen in Fig. 1, a distance d between the first and the second longitudinally extending side sections 16a,16b increases rearwardly, as seen in the longitudinal direction L. The V-shaped section 15v of the embossed front functional design element 15 and the front V-shaped section 16vf of the embossed crotch functional design element 16 are arranged with a distance to each other of between 2 and 20 mm, as seen in the longitudinal direction L.

The first and the second longitudinally extending side sections 16a,16b of the crotch embossed functional design element 16 have a respective first and second rear side section end point 16ar, 16br, the first and second rear side section end point being arranged not more than 15 mm from the first and/or second rear wing junction 18b,19b.

An embossed rear functional design element 17 is arranged in the rear portion 11 of the sanitary article 1. The embossed rear functional design element 17 has a first V-shaped section 17vf pointing rearwardly of the sanitary article 1 and a second V-shaped section 17vr pointing towards the front portion of the sanitary article 1. The second V-shaped section 17vr of the embossed rear functional design element 17 is provided closer to the rear transverse edge 8 of the sanitary article 1 than the first V-shaped section 17vf of the embossed rear functional design element 17. The first and the second V-shaped sections 17vf,17vr of the embossed rear functional design element 17 are connected with a first and a second longitudinally extending side section 17a,17b of the embossed rear functional design element 17.

In contrast to the sanitary article illustrates in Fig. 1, the inner region 17' of the embossed rear functional design element 17 of the Fig. 2 embodiment is provided with a first and a second embossed line 17'a,17'b, wherein at least a section of the respective first and the second embossed lines 17'a,17'b extend in parallel with a respective first and second leg section 17vfa,17vfb of the first V-shaped section 17vf of the embossed rear functional design element 17. The inner region 17' of the embossed rear functional design element 17 furthermore comprises a third embossed line 17'c at least partly extending in parallel with the first leg section 17vfa and a fourth embossed line 17'd at least partly extending in parallel with the second leg section 17vfb.

A tip 16rt of the rear V-shaped section 16vr of the embossed crotch functional design element 16 is arranged with a distance of between 2 mm and 20 mm from a tip of the first V-shaped section 17vf of the embossed rear functional design element 17.

The absorbent core 4 is furthermore provided with a first embossed side line 23, extending along the first longitudinal side edge 5 from the head portion 12, over the neck portion 12 and along a part of the body portion 14, and a second embossed side line 24, extending along the second longitudinal side edge 5 from the head portion 12, over the neck portion 12 and along a part of the body portion 14. The first and second embossed side lines 23,24 being provided with a distance from 3 mm to 10 mm from the respective first and second longitudinal side edge 5,6.

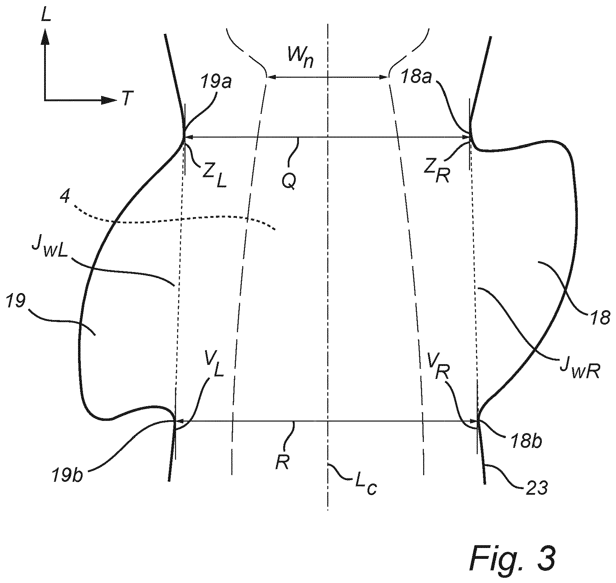

In the context of the present disclosure, the front wing junctions 18a,19a and the rear wing junctions 18b,19b are defined as follows and with reference to Figure 3. In the crotch portion 10, a respective imaginary longitudinal straight line Z_{R} and Z_{L} is drawn, in the longitudinal direction L such that it touches an outer periphery 23 of the sanitary article 1 at the narrowest width Q in the transverse direction of the sanitary article 1 forward of the first and second fastening wing 18, 19.

The intersection between an imaginary straight line z_{R} forward of the first fastening wing 18 and the outer periphery 23 is defined as the first front wing junction 18a, whereas the intersection between the imaginary straight line z_{L} forward of the second fastening wing 19 and the outer periphery 23 is defined as the second front wing junction 19a. In embodiments where the imaginary straight line Z_{R} and Z_{L} coincides with the longitudinal side edges of the sanitary article 1 (e.g. because the longitudinal side edges of the sanitary article consist of straight lines), the front wing junctions 18a and 19a are defined as the point located on the section of the imaginary straight line coinciding with the outer periphery, being most closely located in the forward direction to the wing of the sanitary article. The rear wing junctions 18b, 19b are defined by drawing a respective imaginary longitudinal straight line V_{R} and V_{L}, and such that it touches an outer periphery 26 along a respective longitudinal side edge of the sanitary article 1 3 at the narrowest width R in the transverse direction of the sanitary article 1 rearward of the first and second wing 18, 19. The intersection between the imaginary straight line V_{L} rearward of the second wing 19 and the outer periphery 23 is defined as the second rear wing junction 19b, whereas the intersection between the imaginary straight line V_{R} rearward of the first wing 18 and the outer periphery 23 is defined as the first rear wing junction 19b. In embodiments where the imaginary straight line V_{L}, V_{R} coincides with the longitudinal side edges of the sanitary article 1 (e.g. because the longitudinal side edges of the outer periphery consist of straight lines), the rear wing junctions 18b and 19b are defined as the point located on the section of the imaginary straight line coinciding with the outer periphery 23, being most closely located in the rearward direction to the wing of the sanitary article, i.e. the wing deflection point. As shown in figure 3, the imaginary second wing junction line J_{wL} connects the second front wing junction 19a with the second rear wing junction 19b, while the imaginary first wing junction line J_{wR} connects the first front wing junction 18a with the first rear wing junction 19b. In the context of this application, the first and second wing junction lines J_{wR} and J_{wL} correspond to the wing length. In a sanitary napkin of the type shown, the rear wing junctions 18, 19 are spaced further outboard of a longitudinal centerline L_{C} of the sanitary article 1 as compared to the front wing junctions 18a, 19a. Consequently, the first and second wing junction lines J_{wR} and J_{wL} are not parallel to the longitudinal centerline L_{C}. However, the rear wing junctions 18b,19b may also be spaced such that the transverse distance between the rear wing junctions 18b,19b and the longitudinal centerline L_{C} is equal to the transverse distance between the front wing junctions 18a, 19a and the longitudinal centerline L_{C}. In such an embodiment, the first and second wing junction lines J_{wR} and J_{wL} are parallel to the longitudinal centerline L_{C}. In a sanitary napkin of the type shown, the first and second wing junction lines J_{wR} and J_{wL} have a length of 50-110 mm, preferably between 75-100 mm, and most preferably between 85-95 mm.

## Claims

1. A sanitary article (1), being a sanitary napkin or a pantiliner, the sanitary article comprising a topsheet (2), a backsheet (3) and an absorbent core (4) arranged between the topsheet (2) and the backsheet (3), the sanitary article (1) having an extension in a longitudinal direction (L) and in a transverse direction (T), the sanitary article (1) having first and second longitudinal side edges (5,6) extending in the longitudinal direction (L) and front and rear transverse side edges (7,8) extending in the transverse direction (T), the sanitary article made up of a front portion (9), a crotch portion (10) and a rear portion (11), as seen in the longitudinal direction (L), the absorbent core (4) comprising a head portion (13), a body portion (14) and a neck portion (12), the neck portion forming a transitional region between the head portion (13) and the body portion (14), the head portion (13) being positioned in the front portion (9) of the sanitary article (1) and the body portion (14) being positioned in the crotch portion (10) and in the rear portion (11) of the sanitary article (1), wherein a transverse width (wₙ) of the absorbent core (4) at the neck region (12) is smaller than a maximum transverse width of the absorbent core (4) in the head portion (13) and in the body portion (14),
**characterized in that** the absorbent core (4) comprises three individual and separate embossed functional design elements (15,16,17):
an embossed front functional design element (15) being arranged in the front portion (9) of the sanitary article (1),
an embossed crotch functional design element (16) being arranged in the crotch portion (10) of the sanitary article (1) and
an embossed rear functional design element (17) being arranged in the rear portion (11) of the sanitary article (1),
wherein each of the embossed functional design elements (15;16;17) has a boundary comprising continuous or discontinuous line(s) delimiting a respective inner region (15';16;'17') of the embossed functional design element (15;16;17) respectively and wherein each of the embossed front, crotch and rear functional design elements (15,16,17) is different from one another in shape and/or size.

2. The sanitary article (1) according to claim 1, wherein the sanitary article (1) has a first fastening wing (18) extending along the first longitudinal side edge (5) between a first front wing junction (18a) and a first rear wing junction (18b) and a second fastening wing (19) extending along the second longitudinal side edge (6) between a second front wing junction (19a) and a second rear wing junction (19b).

3. The sanitary article (1) according to claim 2, wherein the first front wing junction (18a) and/or the second front wing junction (19a) are/is aligned with the neck region (12) of the absorbent core (4), as seen in the longitudinal direction (L), or are/is arranged not more than 30 mm, preferably not more than 15 mm, rearwardly from the neck region (12) as measured in the longitudinal direction (L).

4. The sanitary article (1) according to any one of the preceding claims, wherein the embossed front functional design element (15) has a V-shaped section (15v) pointing towards the rear portion (11) of the sanitary article (1) and wherein a tip of the V-shaped section (15v) of the embossed front element (15) is arranged not more than 30 mm, preferably not more than 15 mm, from the neck region (12), as measured in the longitudinal direction (L).

5. The sanitary article according to any one of the preceding claims, wherein the embossed crotch functional design element (16) comprises a first and a second longitudinally extending side section (16a,16b) and where a distance (d) between the first and the second longitudinally extending side sections (16a, 16b) increases rearwardly, as seen in the longitudinal direction (L).

6. The sanitary article according to any one of the preceding claims, wherein the embossed crotch functional design element (16) has a front V-shaped section (16vf) pointing towards the front edge (7) of the sanitary article (1) and/or a rear V-shaped section (16vf) pointing towards the rear edge (8) of the sanitary article (1).

7. The sanitary article according to claim 5 and 6, wherein the front and/or rear V-shaped sections (16vf,16vr) of the embossed crotch functional design element (16) are connected to the first and second longitudinally extending side sections (16a,16b).

8. The sanitary article according to claim 4 and either of claim 6 and 7, wherein the V-shaped section (15v) of the embossed front functional design element (15) and the front V-shaped section (16vf) of the embossed crotch functional design element (16) are arranged with a distance to each other of between 2 and 15 mm, as seen in the longitudinal direction (L).

9. The sanitary article according to claim 2 or 3 in combination with either of claim 5 and 8, wherein the first and the second longitudinally extending side sections (16a,16b) of the crotch embossed functional design element (16) have a respective first and second rear side section end point (16ar, 16br) and wherein the first and/or second rear side section end point are/is aligned with or are/is arranged not more than 30 mm from the first and/or second rear wing junction (18b,19b).

10. The sanitary article according to any one of the preceding claims, wherein the embossed rear functional design element (17) has a first V-shaped section (17vf) pointing rearwardly of the sanitary article (1).

11. The sanitary article according to claim 10, wherein the embossed rear functional design element (17) has a second V-shaped section (17vr) pointing towards the front portion of the sanitary article (1) and wherein the second V-shaped section is provided closer to the rear transverse edge (8) of the sanitary article (1) than the first V-shaped section (17vf) of the embossed rear functional design element (17) and wherein the first and the second V-shaped sections (17vf, 17vr) of the embossed rear functional design element (17) are connected with a first and a second longitudinally extending side section (17a,17b) of the embossed rear functional design element (17).

12. The sanitary article according to claim 10 or 11, wherein the inner region (17') of the embossed rear functional design element is provided with a first and a second embossed line (17'a, 17'b) and wherein the first and the second embossed line extend in parallel with a respective leg section (17vfa, 17vfb) of the first V-shaped section (17vf) of the embossed rear functional design element (17).

13. The sanitary article according to claim 6 and claim 9 or 10, wherein a tip (16rt) of the rear V-shaped section (16vr) of the embossed crotch functional design element (16) is arranged at a distance of between 2 mm and 20 mm from a tip of the first V-shaped section (17vf) of the embossed rear functional design element (17), optionally wherein a first and a second leg section of the rear V-shaped section (16vra,16vrb) of the embossed crotch functional design element (16) extend in parallel with a first and a second leg section (17vra, 17vrb) of the first V-shaped section (17vr) of the embossed rear functional design element (17).

14. The sanitary article according to any one of the preceding claims, wherein each of the embossed front functional design element (15), the embossed crotch functional design element (16) and the embossed rear functional design element (17) is aligned with each other, as seen in the longitudinal direction (L), and centred along a longitudinal centerline (L_{C}) of the sanitary article (1).

## Patentansprüche

1. Hygieneartikel (1), bei dem es sich um eine Damenbinde oder eine Slipeinlage handelt, wobei der Hygieneartikel eine Oberschicht (2), eine Unterschicht (3) und einen absorbierenden Kern (4) umfasst, der zwischen der Oberschicht (2) und der Unterschicht (3) angeordnet ist, wobei der Hygieneartikel (1) eine Ausdehnung in Längsrichtung (L) und in Querrichtung (T) aufweist, wobei der Hygieneartikel (1) erste und zweite Längsseitenränder (5,6), die sich in Längsrichtung (L) erstrecken, und vordere und hintere Querseitenränder (7,8) aufweist, die sich in Querrichtung (T) erstrecken, wobei der Hygieneartikel in Längsrichtung (L) gesehen aus einem vorderen Teil (9), einem Schrittteil (10) und einem hinteren Teil (11) besteht, wobei der absorbierende Kern (4) einen Kopfteil (13), einen Körperteil (14) und einen Halsteil (12) umfasst, wobei der Halsteil einen Übergangsbereich zwischen dem Kopfteil (13) und dem Körperteil (14) bildet, wobei der Kopfteil (13) in dem vorderen Teil (9) des Hygieneartikels (1) angeordnet ist und der Körperteil (14) in dem Schrittteil (10) und in dem hinteren Teil (11) des Hygieneartikels (1) angeordnet ist, wobei eine Querbreite (wₙ) des absorbierenden Kerns (4) in dem Halsbereich (12) kleiner als eine maximale Querbreite des absorbierenden Kerns (4) in dem Kopfteil (13) und in dem Körperteil (14) ist, **dadurch gekennzeichnet, dass** der absorbierende Kern (4) drei einzelne und separate geprägte Funktionsdesignelemente (15,16,17) umfasst:
ein geprägtes vorderes Funktionsdesignelement (15), das in dem vorderen Teil (9) des Hygieneartikels (1) angeordnet ist,
ein geprägtes Schritt-Funktionsdesignelement (16), das in dem Schrittteil (10) des Hygieneartikels (1) angeordnet ist, und
ein geprägtes hinteres Funktionsdesignelement (17), das in dem hinteren Teil (11) des Hygieneartikels (1) angeordnet ist,
wobei jedes der geprägten Funktionsdesignelemente (15;16;17) eine Begrenzung aufweist, die eine oder mehrere durchgehende oder unterbrochene Linie(n) umfasst, die einen jeweiligen inneren Bereich (15';16;'17') des geprägten Funktionsdesignelements (15;16;17) begrenzen, und wobei sich jedes der geprägten Funktionsdesignelemente (15,16,17) vorne, im Schritt und hinten in Form und/oder Größe voneinander unterscheidet.

2. Hygieneartikel (1) nach Anspruch 1, wobei der Hygieneartikel (1) einen ersten Befestigungsflügel (18), der sich entlang der ersten Längsseitenkante (5) zwischen einer ersten vorderen Flügelverbindung (18a) und einer ersten hinteren Flügelverbindung (18b) erstreckt, und einen zweiten Befestigungsflügel (19) aufweist, der sich entlang der zweiten Längsseitenkante (6) zwischen einer zweiten vorderen Flügelverbindung (19a) und einer zweiten hinteren Flügelverbindung (19b) erstreckt.

3. Hygieneartikel (1) nach Anspruch 2, wobei die erste vordere Flügelverbindung (18a) und/oder die zweite vordere Flügelverbindung (19a) in Längsrichtung (L) gesehen mit dem Halsbereich (12) des absorbierenden Kerns (4) ausgerichtet sind/ist, oder in Längsrichtung (L) gemessen nicht mehr als 30 mm, vorzugsweise nicht mehr als 15 mm, hinter dem Halsbereich (12) angeordnet sind/ist.

4. Hygieneartikel (1) nach einem der vorhergehenden Ansprüche, wobei das geprägte vordere Funktionsdesignelement (15) einen V-förmigen Abschnitt (15v) aufweist, der in Richtung des hinteren Teils (11) des Hygieneartikels (1) zeigt, und wobei eine Spitze des V-förmigen Abschnitts (15v) des geprägten vorderen Elements (15) in Längsrichtung (L) gemessen nicht mehr als 30 mm, vorzugsweise nicht mehr als 15 mm, von dem Halsbereich (12) angeordnet ist.

5. Hygieneartikel nach einem der vorhergehenden Ansprüche, wobei das geprägte Schritt-Funktionsdesignelement (16) einen ersten und einen zweiten sich in Längsrichtung erstreckenden Seitenabschnitt (16a,16b) umfasst und wobei ein Abstand (d) zwischen dem ersten und dem zweiten sich in Längsrichtung erstreckenden Seitenabschnitt (16a,16b) in Längsrichtung (L) gesehen nach hinten zunimmt.

6. Hygieneartikel nach einem der vorhergehenden Ansprüche, wobei das geprägte Schritt-Funktionsdesignelement (16) einen vorderen V-förmigen Abschnitt (16vf), der in Richtung der vorderen Kante (7) des Hygieneartikels (1) zeigt, und/oder einen hinteren V-förmigen Abschnitt (16vf) aufweist, der in Richtung der hinteren Kante (8) des Hygieneartikels (1) zeigt.

7. Hygieneartikel nach Anspruch 5 und 6, wobei der vordere und/oder hintere V-förmige Abschnitt (16vf, 16vr) des geprägten Schritt-Funktionsdesignelements (16) mit dem ersten und zweiten sich in Längsrichtung erstreckenden Seitenabschnitt (16a,16b) verbunden sind.

8. Hygieneartikel nach Anspruch 4 und einem der Ansprüche 6 oder 7, wobei der V-förmige Abschnitt (15v) des geprägten vorderen Funktionsdesignelements (15) und der vordere V-förmige Abschnitt (16vf) des geprägten Schritt-Funktionsdesignelements (16) in Längsrichtung (L) gesehen in einem Abstand zwischen 2 und 15 mm zueinander angeordnet sind.

9. Hygieneartikel nach Anspruch 2 oder 3 in Kombination mit einem der Ansprüche 5 oder 8, wobei der erste und der zweite sich in Längsrichtung erstreckende Seitenabschnitt (16a,16b) des geprägten Schritt-Funktionsdesignelements (16) jeweils einen ersten und zweiten Endpunkt des hinteren Seitenabschnitts (16ar,16br) aufweisen und wobei der erste und/oder zweite Endpunkt des hinteren Seitenabschnitts mit nicht mehr als 30 mm von der ersten und/oder zweiten hinteren Flügelverbindung (18b,19b) ausgerichtet sind/ist oder angeordnet sind/ist.

10. Hygieneartikel nach einem der vorhergehenden Ansprüche, wobei das geprägte hintere Funktionsdesignelement (17) einen ersten V-förmigen Abschnitt (17vf) aufweist, der nach hinten von dem Hygieneartikel (1) zeigt.

11. Hygieneartikel nach Anspruch 10, wobei das geprägte hintere Funktionsdesignelement (17) einen zweiten V-förmigen Abschnitt (17vr) aufweist, der in Richtung des vorderen Teils des Hygieneartikels (1) zeigt, und wobei der zweite V-förmige Abschnitt näher an der hinteren Querkante (8) des Hygieneartikels (1) als der erste V-förmige Abschnitt (17vf) des geprägten hinteren Funktionsdesignelements (17) vorgesehen ist und wobei der erste und der zweite V-förmige Abschnitt (17vf,17vr) des geprägten hinteren Funktionsdesignelements (17) mit einem ersten und einem zweiten sich in Längsrichtung erstreckenden Seitenabschnitt (17a, 17b) des geprägten hinteren Funktionsdesignelements (17) verbunden sind.

12. Hygieneartikel nach Anspruch 10 oder 11, wobei der innere Bereich (17') des geprägten hinteren Funktionsdesignelements mit einer ersten und einer zweiten Prägelinie (17'a,17'b) versehen ist und wobei sich die erste und die zweite Prägelinie parallel zu einem jeweiligen Beinabschnitt (17vfa,17vfb) des ersten V-förmigen Abschnitts (17vf) des geprägten hinteren Funktionsdesignelements (17) erstrecken.

13. Hygieneartikel nach Anspruch 6 und Anspruch 9 oder 10, wobei eine Spitze (16rt) des hinteren V-förmigen Abschnitts (16vr) des geprägten Schritt-Funktionsdesignelements (16) in einem Abstand zwischen 2 mm und 20 mm von einer Spitze des ersten V-förmigen Abschnitts (17vf) des geprägten hinteren Funktionsdesignelements (17) angeordnet ist, wobei sich optional ein erster und ein zweiter Beinabschnitt des hinteren V-förmigen Abschnitts (16vra,16vrb) des geprägten Schritt-Funktionsdesignelements (16) parallel zu einem ersten und einem zweiten Beinabschnitt (17vra,17vrb) des ersten V-förmigen Abschnitts (17vr) des geprägten hinteren Funktionsdesignelements (17) erstrecken.

14. Hygieneartikel nach einem der vorhergehenden Ansprüche, wobei das geprägte vordere Funktionsdesignelement (15), das geprägte Schritt-Funktionsdesignelement (16) und das geprägte hintere Funktionsdesignelement (17) jeweils in Längsrichtung (L) gesehen miteinander ausgerichtet sind und entlang einer Längsmittellinie (L_{c}) des Hygieneartikels (1) zentriert sind.

## Revendications

1. Article d'hygiène (1), étant une serviette hygiénique ou un protège-slip, l'article d'hygiène comprenant une feuille supérieure (2), une feuille inférieure (3) et un noyau absorbant (4) disposé entre la feuille supérieure (2) et la feuille inférieure (3), l'article d'hygiène (1) ayant une extension dans une direction longitudinale (L) et dans une direction transversale (T), l'article d'hygiène (1) ayant un premier et deuxième bord latéraux longitudinaux (56) s'étendant dans la direction longitudinale (L) et des bords latéraux transversaux avant et arrière (7, 8) s'étendant dans la direction transversale (T), l'article d'hygiène étant constitué d'une partie avant (9), d'une partie d'entrejambe (10) et d'une partie arrière (11), vues dans la direction longitudinale (L), le noyau absorbant (4) comprenant une partie de tête (13), une partie de corps (14) et une partie de col (12), la partie de col formant une zone de transition entre la partie de tête (13) et la partie de corps (14), la partie de tête (13) étant positionnée dans la partie avant (9) de l'article d'hygiène (1) et la partie de corps (14) étant positionnée dans la partie d'entrejambe (10) et dans la partie arrière (11) de l'article d'hygiène (1), où une largeur transversale (wₙ) du noyau absorbant (4) au niveau de la zone de col (12) est inférieure à une largeur transversale maximale du noyau absorbant (4) dans la partie de tête (13) et dans la partie de corps (14),
**caractérisé en ce que** le noyau absorbant (4) comprend trois éléments gaufrés de conception fonctionnelle individuels et séparés (15, 16, 17) :
un élément gaufré de conception fonctionnelle avant (15) étant disposé dans la partie avant (9) de l'article d'hygiène (1),
un élément gaufré de conception fonctionnelle d'entrejambe (16) étant disposé dans la partie d'entrejambe (10) de l'article d'hygiène (1) et
un élément gaufré de conception fonctionnelle arrière (17) étant disposé dans la partie arrière (11) de l'article d'hygiène (1),
où chacun des éléments gaufrés de conception fonctionnelle (15 ; 16 ; 17) présente une limite comprenant une ou plusieurs lignes continues ou discontinues délimitant une zone intérieure respective (15' ; 16' ; 17') de l'élément gaufré de conception fonctionnelle (15 ; 16 ; 17) respectivement, et où chacun des éléments gaufrés de conception fonctionnelle avant, d'entrejambe et arrière (15, 16, 17) est différent les uns des autres en termes de forme et/ou de taille.

2. Article d'hygiène (1) selon la revendication 1, où l'article d'hygiène (1) a une première ailette de fixation (18) s'étendant le long du premier bord latéral longitudinal (5) entre une jonction avant de première ailette (18a) et une jonction arrière de première ailette (18b) et une deuxième ailette de fixation (19) s'étendant le long du deuxième bord latéral longitudinal (6) entre une jonction avant de deuxième ailette (19a) et une jonction arrière de deuxième ailette (19b).

3. Article d'hygiène (1) selon la revendication 2, où la jonction avant de première ailette (18a) et/ou la jonction avant de deuxième ailette (19a) sont/est alignée(s) avec la zone de col (12) du noyau absorbant (4), vue dans la direction longitudinale (L), ou sont/est disposée(s) à une distance maximale de 30 mm, de préférence pas plus de 15 mm, vers l'arrière de la zone de col (12), telle que mesurée dans la direction longitudinale (L).

4. Article d'hygiène (1) selon l'une quelconque des revendications précédentes, où l'élément gaufré de conception fonctionnelle avant (15) présente une section en forme de V (15v) pointant vers la partie arrière (11) de l'article d'hygiène (1) et où une pointe de la section en forme de V (15v) de l'élément gaufré avant (15) est disposée à une distance maximale de 30 mm, de préférence pas plus de 15 mm, de la zone de col (12), telle que mesurée dans la direction longitudinale (L).

5. Article d'hygiène selon l'une quelconque des revendications précédentes, où l'élément gaufré de conception fonctionnelle d'entrejambe (16) comprend une première et une deuxième section latérale s'étendant longitudinalement (16a, 16b) et où une distance (d) entre la première et la deuxième section latérale s'étendant longitudinalement (16a, 16b) augmente vers l'arrière, vues dans la direction longitudinale (L).

6. Article d'hygiène selon l'une quelconque des revendications précédentes, où l'élément gaufré de conception fonctionnelle d'entrejambe (16) a une section avant en forme de V (16vf) pointant vers le bord avant (7) de l'article d'hygiène (1) et/ou une section arrière en forme de V (16vf) pointant vers le bord arrière (8) de l'article d'hygiène (1).

7. Article d'hygiène selon les revendications 5 et 6, où les sections avant et/ou arrière en forme de V (16vf, 16vr) de l'élément gaufré de conception fonctionnelle d'entrejambe (16) sont reliées à la première et à la deuxième section latérale s'étendant longitudinalement (16a, 16b).

8. Article d'hygiène selon la revendication 4 et l'une ou l'autre des revendications 6 et 7, où la section en forme de V (15v) de l'élément gaufré de conception fonctionnelle avant (15) et la section avant en forme de V (16v) de l'élément gaufré de conception fonctionnelle d'entrejambe (16) sont disposées à une distance l'une de l'autre comprise entre 2 et 15 mm, vues dans la direction longitudinale (L).

9. Article d'hygiène selon la revendication 2 ou 3 en combinaison avec l'une des revendications 5 et 8, où la première et deuxième section latérale s'étendant longitudinalement (16a, 16b) de l'élément gaufré de conception fonctionnelle d'entrejambe (16) ont un premier et un deuxième point d'extrémité de section latérale arrière (16ar, 16br) respectif et où le premier et/ou le deuxième point d'extrémité de section latérale arrière sont/est aligné(s) avec ou sont/est disposé(s) à une distance maximale de 30 mm de la jonction arrière de première et/ou de deuxième ailette (18b, 19b).

10. Article d'hygiène selon l'une quelconque des revendications précédentes, où l'élément gaufré de conception fonctionnelle arrière (17) a une première section en forme de V (17vf) pointant vers l'arrière de l'article d'hygiène (1).

11. Article d'hygiène selon la revendication 10, où l'élément gaufré de conception fonctionnelle arrière (17) a une deuxième section en forme de V (17vr) pointant vers la partie avant de l'article d'hygiène (1) et où la deuxième section en forme de V est prévue plus près du bord transversal arrière (8) de l'article d'hygiène (1) que la première section en forme de V (17vf) de l'élément gaufré de conception fonctionnelle arrière (17) et où la première et deuxième section en forme de V (17vf, 17vr) de l'élément gaufré de conception fonctionnelle arrière (17) sont reliées à une première et à une deuxième section latérale s'étendant longitudinalement (17a, 17b) de l'élément gaufré de conception fonctionnelle arrière (17).

12. Article d'hygiène selon la revendication 10 ou 11, où la zone intérieure (17') de l'élément gaufré de conception fonctionnelle arrière est pourvue d'une première et d'une deuxième ligne gaufrée (17'a, 17'b) et où la première et la deuxième ligne gaufrée s'étendent parallèlement à une section de jambe respective (17vfa, 17vfb) de la première section en forme de V (17vf) de l'élément gaufré de conception fonctionnelle arrière (17).

13. Article d'hygiène selon la revendication 6 et la revendication 9 ou 10, où une pointe (16rt) de la section arrière en forme de V (16vr) de l'élément gaufré de conception fonctionnelle d'entrejambe (16) est disposée à une distance comprise entre 2 mm et 20 mm d'une pointe de la première section en forme de V (17vf) de l'élément gaufré de conception fonctionnelle arrière (17), où, éventuellement, une première et une deuxième section de jambe de la section arrière en forme de V (16vra, 16vrb) de l'élément gaufré de conception fonctionnelle d'entrejambe (16) s'étendent parallèlement à une première et à une deuxième section de jambe (17vra, 17vrb) de la première section en forme de V (17vr) de l'élément gaufré de conception fonctionnelle arrière (17).

14. Article d'hygiène selon l'une quelconque des revendications précédentes, où chacun des éléments gaufrés de conception fonctionnelle avant (15), des éléments gaufrés de conception fonctionnelle d'entrejambe (16) et des éléments gaufrés de conception fonctionnelle arrière (17) est aligné les uns avec les autres, vus dans la direction longitudinale (L), et centré le long d'une ligne médiane longitudinale (L_{c}) de l'article d'hygiène (1).
